# EUROPEAN PATENT APPLICATION

(11) **EP 4 129 243 A1**
(43) Date of publication of application: **08.02.2023**
(21) Application number: 21776279.8
(22) Date of filing: 24.03.2021
(51) Int. Cl.: A61F 2/95

(54) **STENT ASSEMBLY SYSTEM AND STENT ASSEMBLY METHOD**

(30) Priority: 26.03.2020 CN 202010225221
(71) Applicant: Shenzhen Lifetech Endovascular Medical Co., Ltd., Shenzhen, Guangdong 518000 (CN)
(72) Inventor: PU, Wenjun, Shenzhen, Guangdong 518000 (CN); XIAO, Benhao, Shenzhen, Guangdong 518000 (CN); LIU, Qiang, Shenzhen, Guangdong 518000 (CN); LING, Zhengqing, Shenzhen, Guangdong 518000 (CN)
(74) Representative: Prinz & Partner mbB
(86) International application number: PCT/CN2021/082678
(87) International publication number: WO 2021/190549

(57) **Abstract**

A stent assembly system and a stent assembly method are provided. The stent assembly system includes a stent and an assembly instrument; the assembly instrument is configured to assemble and deliver the stent, and includes a sheath core tube, an outer sheath tube, and an assembly part; the outer sheath tube slidably surrounds the sheath core tube in an axial direction; an accommodating cavity for accommodating the stent is formed between the inner wall of the outer sheath tube and the outer wall of the sheath core tube; the assembly part has a fixed end and a free end opposite to the fixed end, and the fixed end is connected to the sheath core tube; and when the stent is crimped radially on the sheath core tube, the free end is hooked to the stent to limit the stent.

## Description

### Technical Field

The present invention relates to the field of medical devices, and more particularly relates to a stent assembly system and a stent assembly method.

### Background Art

This section provides only background information related to the present utility model, which is not necessarily the existing art.

Interventional therapy has the characteristics of less trauma and fewer complications. At present, the treatment of cardiovascular diseases by interventional therapy has become a common treatment method. With the continuous development of interventional technology and the advancement of the interventional therapy technology, the advantages of using interventional technology to treat aneurysm, arterial dissection disease, vascular stenosis, and other cardiovascular diseases have become increasingly prominent. In interventional technology, stents are common devices. According to the classification of structures, stents are mainly divided into two categories. One type is a covered stent, which is mainly composed of a covered film and a metal stent supporting the covered film. The covered film is generally made of polyester, an expanded polytetrafluoroethylene (e-PTFE) film, and other materials with good biocompatibility, and the metal stent is generally made of nickel-titanium alloy stent or stainless steel. The other type is a bare stent, which does not contain a covered film and generally consists of a metal stent such as a stainless steel stent or a nickel-titanium alloy stent. The metal stents in the covered stent and the bare stent can be classified into a woven metal stent and a cut metal stent according to the preparation method. A raw material of the woven metal stent is generally a metal wire rod, and the woven metal stent is formed by braiding and heat-setting. A raw material of the cut metal stent is generally a tube, and the cut metal stent is formed by cutting and heat-setting.

Whether it is a covered stent or a bare stent, in interventional therapy, the stent is pre-assembled in a sheath tube of a delivery system. During the operation, the delivery system delivers the stent to the location of a lesion and releases the stent. The stent expands and is secured to a vascular wall to isolate blood flow from the aneurysm and the arterial dissection, or to reopen a narrowed vascular channel, so as to re-establish a channel for normal blood circulation and achieve the interventional treatment of the aneurysm, the arterial dissection or arterial stenosis and other diseases.

With the wide application of the stent intervention technology, more and more clinical problems need to be optimized. Problems such as the inability to release the stent, the distortion of a stent release shape, damage to the stent, and the like, are more common. The inability to release the stent leads to a failure in achieving the interventional therapy. The distortion of the shape after the stent release may lead to problems of poor positioning of the stent, and damage to the stent may lead to insufficient radial support and other problems, thus affecting the treatment effect. In order to solve these problems, at present, many research efforts focus on a release mechanism, while the influence of the accuracy of the assembly on whether the stent can be released accurately is ignored. The optimization of the release mechanism is conducive to improving the release accuracy, and the accuracy of the assembly also plays a crucial role in the release accuracy. In the process of assembling the stent to the sheath tube, the stacking or twisting of the stent will adversely affect the release accuracy of the stent, and in severe cases, the stent may not be released.

At present, there are two primary ways to assemble a stent. One method is that a radial force applied to the stent causes the stent to be crimped on a sheath core tube, and then the stent is pushed into the sheath tube together with the sheath core tube. In this assembly method, the stent is subjected to a radial compression force and an axial push force, and the axial force push force pushes the stent and sheath core tube into the sheath tube together. The relative sliding between the stent and the sheath core tube in the pushing process will cause a phenomenon called stacking, which will affect the accuracy of assembly of the stent. The stacking caused by this type of assembly method generally includes the following three situations: 1) Stacking occurs at or near a proximal end. This is because, in the early stage of assembly, when the relative sliding between the stent and the sheath core tube occurs, an axial displacement of the stent is not completely synchronized with that of the sheath core tube. When the amount of axial displacement of the stent is greater than that of the sheath core tube, it will cause the proximal part of the stent to enter the sheath tube, while the corresponding sheath core tube part is still located outside the sheath tube. Under the action of the continuous axial pushing force, the part of the stent that has been assembled into the sheath tube begins to move towards the proximal end, and finally, wave loop stacking occurs at or near the proximal end. 2) Stacking occurs in the middle of the stent. During the middle of assembly, the stent and the sheath core tube slide relative to each other, so that when the stent is pushed into the sheath tube, the corresponding sheath core tube is not completely pushed into the sheath tube. Furthermore, the part of the stent that has been assembled into the sheath tube and the sheath core tube part do not move relative to each other. Under the action of the continuous axial pushing force, the phenomenon of stacking in the middle of the stent occurs. 3) Stacking occurs at or near a distal end. During the initial assembly process, the stent and the sheath core tube slide relative to each other, so that the axial displacement amount of the stent towards the sheath tube is less than the corresponding axial displacement amount of the sheath core. Therefore, after the sheath core tube part is pushed into the sheath tube, the corresponding part of the stent is still located outside the sheath tube, so that during the final stage of assembly, there are more parts that are not assembled into the sheath tube. In this way, continuous assembling will cause the stacking of the stent at or near the distal end.

In addition, due to different requirements of different implant sites for the performance of the stent, in design, circumferential waveforms of many stents have different heights, wave angles and/or metal amounts. Therefore, when the stent is subjected to a radial compression force, the counter-reacting forces which are generated by all circumferential parts of the stent and which are opposite to the radial compression force are different. The counter-reacting forces which are generated by all circumferential parts of the stent are not necessarily uniformly distributed in the circumferential direction. Some waveforms generate a low counter-reacting force, while others generate a high counter-reacting force. The amount of compression of the waveform with the low counter-reacting force is significantly greater than that of the waveform with the high counter-reacting force. During the assembly process, before entering the sheath tube, the uniformity of the compression of the stent can be controlled and adjusted artificially, but after entering the sheath tube, the trend that the stent returns to its circumferential non-uniform distribution is unavoidable. Thus, the waveform after assembly is shifted, i.e., twisted, from its phase before the stent is pushed into the sheath tube. In addition, during the process of pushing the stent and the sheath core tube into the sheath tube, the circumferential movement of the stent relative to the sheath core tube possibly makes the stent twisted. This will cause the stent to assume a twisted shape after it has been assembled into the sheath tube. Thus, the stent will also be twisted when it is implanted and released. In the case of severe twisting, the stent may be even damaged.

Another way to assemble the stent is to radially press the stent on the sheath core tube, keep the stent and the sheath core tube in the same position, and then apply a pushing force to the sheath tube to cause the sheath tube to move towards the stent and the sheath core tube, so as to assemble the stent and the sheath core tube into an inner cavity of the sheath tube. The risks of this assembly method are similar to those of the aforementioned method, and will not be repeated here.

### Summary

Based on the above, it is necessary to provide a stent assembly system and a stent assembly method capable of improving the accuracy of assembly.

A stent assembly system includes a stent and an assembly instrument. The assembly instrument is used to assemble and deliver the stent. The assembly instrument includes a sheath core tube, an outer sheath tube and an assembly part. The outer sheath tube slidably surrounds the sheath core tube in an axial direction, and an accommodating cavity for accommodating the stent is formed between the inner wall of the outer sheath tube and the outer wall of the sheath core tube. The assembly part has a fixed end and a free end opposite to the fixed end. The fixed end is connected to the sheath core tube. When the stent is radially crimped on the sheath core tube, the free end is hooked to the stent to limit the stent.

The sheath core tube of the assembly instrument is provided with the assembly part having the free end. When the stent is radially crimped on the sheath core tube, the free end of the assembly part is hooked to the stent to limit the stent. Thus, in the assembly process, the phenomenon of stacking of the stent caused by relative sliding between the stent and the sheath core tube can be avoided. Therefore, the stent assembly system can improve the accuracy of assembly.

### Brief Description of the Drawings

Fig. 1 is a schematic structural diagram of a stent assembly system according to one embodiment;
Fig. 2 is a schematic structural diagram of an assembly instrument according to one embodiment;
Fig. 3 is a partially schematic diagram of a stent assembly system of one embodiment in an assembly state;
Fig. 4 is a partially schematic diagram of a stent assembly system of another embodiment in an assembly state;
Fig. 5a to Fig. 5d are schematic diagrams of an assembly process of a stent assembly system according to one embodiment;
Fig. 6 is a partially schematic diagram of a stent assembly system of another embodiment in an assembly completed state;
Fig. 7 is a partially schematic structural diagram of an assembly instrument according to another embodiment;
Fig. 8 is a schematic structural diagram of an assembly part according to one embodiment;
Fig. 9 is a schematic diagram of a bent state of an assembly instrument of one embodiment;
Fig. 10 is a schematic structural diagram of an assembly part according to another embodiment;
Fig. 11 is a schematic structural diagram of an assembly part according to another embodiment;
Fig. 12 is a schematic structural diagram of an assembly part according to another embodiment;
Fig. 13 is a schematic structural diagram of an assembly part according to another embodiment;
Fig. 14 is a schematic structural diagram of an assembly part according to another embodiment;
Fig. 15 is a schematic structural diagram of an assembly part according to another embodiment;
Fig. 16 is a partial schematic diagram of a stent assembly system of another embodiment in an assembly completed state;
Fig. 17 is a schematic structural diagram of a stent according to one embodiment;
Fig. 18 is a schematic diagram illustrating that the stent shown in Fig. 17 is in an assembly completed state;
Fig. 19 is a schematic diagram of an assembly part and an outer sheath tube according to one embodiment;
Fig. 20 is a schematic structural diagram of an assembly part according to another embodiment;
Fig. 21 is a schematic structural diagram of a stent according to another embodiment;
Fig. 22 is a schematic diagram of a screenshot along the 1--1 line of Fig. 21;
Fig. 23 and Fig. 24 are schematic diagrams of a cross-section of a stent according to one embodiment in a compressed state;
Fig. 25 is a schematic structural diagram of a woven form of a stent according to one embodiment;
Fig. 26 is a schematic structural diagram of a covered film of a stent according to one embodiment;
Fig. 27 is a schematic structural diagram of a covered film of a stent according to one embodiment;
Fig. 28 is a schematic structural diagram of a covered film of a stent according to one embodiment;
Fig. 29 is a schematic structural diagram of a stent according to another embodiment;
Fig. 30a to Fig. 30e are schematic diagrams showing the release process of a stent assembly system according to one embodiment;
Fig. 31 is a schematic structural diagram of an assembly component according to one embodiment;
Fig. 32 is a schematic structural diagram of an assembly component according to another embodiment; and
Fig. 33 is a schematic structural diagram of an assembly component according to another embodiment.

### Detailed Description

In order to make the foregoing objectives, features and advantages of the present disclosure more obvious and understandable, the specific embodiments of the present disclosure are described in detail with reference to the accompanying drawings. Many specific details are described in the following descriptions to facilitate full understanding of the present disclosure. However, the present disclosure can be implemented in a variety of other ways than those described herein, and those skilled in the art can make similar improvements without departing from the connotation of the present disclosure. Therefore, the present disclosure is not limited by specific embodiments disclosed below.

Unless otherwise defined, all technical and scientific terms used herein are the same as meanings of general understandings of those skilled in the art of the disclosure. The terms used in the description of the disclosure herein are merely to describe the specific embodiments, not intended to limit the disclosure.

In the field of interventional medical apparatuses, "distal end" is defined as an end far from an operator during surgery, and "proximal end" is defined as an end close to the operator during surgery. "Axial" refers to a direction parallel to a connecting line between the center of a distal end of a medical apparatus and the center of a proximal end, and "radial" refers to a direction perpendicular to the above axial direction.

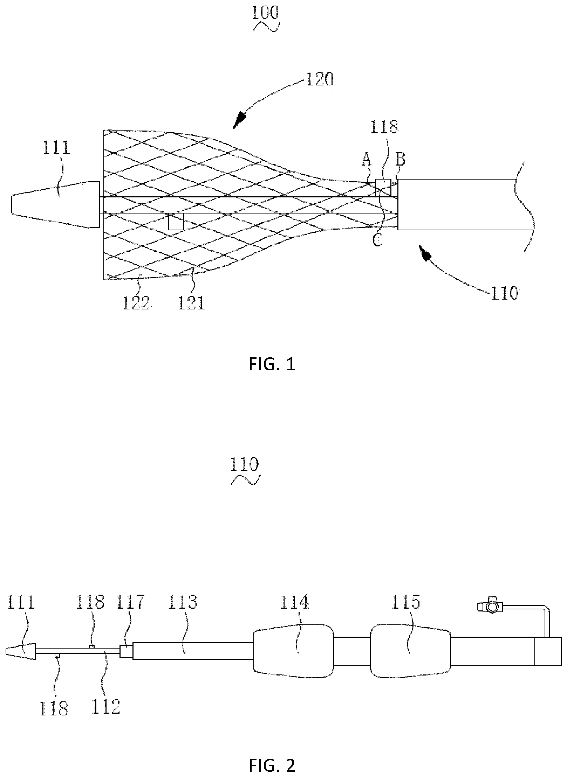

Referring to Fig. 1, a stent assembly system 100 of one embodiment includes an assembly instrument 110 and a stent 120. The assembly instrument 110 is used to assemble the stent 120 and deliver the stent 120 to a lesion site.

Referring to Fig. 2, the assembly instrument 110 includes a guide head 111, a sheath core tube 112, an outer sheath tube 113, a fixed handle 114, and a movable handle 115.

The guide head 111 has a hollow cavity structure having openings in two ends and a conical distal end. The sheath core tube 112 is a hollow tube piece. The distal end of the sheath core tube 112 extends into the guide head 111 from the open end of the proximal end of the guide head 111 and is fixedly connected to the guide head 111. A cavity of the sheath core tube 112 is communicated with a chamber of the guide head 111 to form a guide wire channel, so as to ensure that the assembly instrument 110 can successfully reach the lesion site under the guidance of a guide wire.

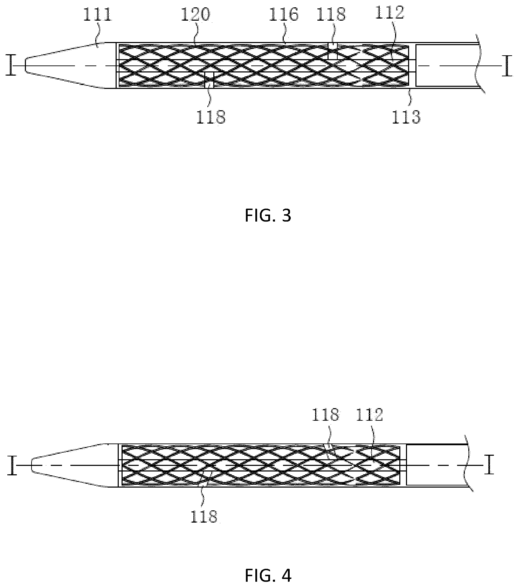

The sheath core tube 112 and the outer sheath tube 113 are both hollow tubular members. The outer sheath tube 113 is coaxial with and surrounds the sheath core tube 112. Furthermore, the outer sheath tube 113 is axially slidable relative to the sheath core tube 112. As shown in Fig. 3, when the outer sheath tube 113 slides to the distal end of the outer sheath tube 113 in the axial direction and resists against or is close to the guide head 111, the outer sheath tube 113 and the sheath core tube 112 are encircled to form a ring accommodating cavity 116, and the stent 120 is accommodated in the accommodating cavity 116. Referring to Fig. 2 and Fig. 3, the fixed handle 114 and the sheath core tube 112 are kept relatively fixed. The movable handle 115 is fixedly connected to the outer sheath tube 113. During the surgical procedure, the fixed handle 114 is held with one hand, and the movable handle 115 is operated with the other hand, so as to cause the outer sheath tube 113 to move in the axial direction towards the proximal end, thus releasing the stent 120.

In one embodiment, as shown in Fig. 2, the assembly instrument 110 further includes a supporting tube 117. The supporting tube 117 surrounds the proximal end of the sheath core tube 112. The supporting tube 117 is coaxial with and fixedly connected to the sheath core tube 112. The outer sheath tube 113 surrounds the supporting tube 117 and is axially slidable with respect to the sheath core tube 112 along the supporting tube 117, or is axially slidable to the supporting tube 117 along the sheath core tube 112. With the arrangement of the supporting tube 117, on the one hand, the distal end of the supporting tube 117 can abut against the stent 120 to perform a limiting function. On the other hand, the supporting tube 117 can better support the outer sheath tube 113 in a region of the outer sheath tube 113 that does not cover the stent 120, so as to prevent the outer sheath tube 113 from bending.

Referring back to Fig. 1 and Fig. 2, the assembly instrument 110 further includes an assembly part 118. The assembly part 118 is used for assisting in the assembly of the stent 120, so as to improve the accuracy of the assembly. The assembly part 118 is arranged on the outer wall of the sheath core tube 112 and is fixedly connected to the sheath core tube 112. The assembly part 118 has a fixed end and a free end. The end connected to the sheath core tube 112 is the fixed end, and the other end opposite to the fixed end is the free end. In one embodiment, the assembly part 118 is a protrusion arranged on the outer wall of the sheath core tube 112, and the end of the assembly part 118 away from the outer wall of the sheath core tube 112 is the free end. In one embodiment, the assembly part 118 is a cylindrical protrusion fixed on the outer wall of the sheath core tube 112. One bottom surface of the cylindrical protrusion is an end surface of the fixed end, and the other bottom surface of the cylindrical protrusion is an end surface of the free end. It can be understood that the shape of the assembly part 118 is not limited. For example, it can be a cylindroid, a square column, and the like.

The stent 120 has a tubular cavity structure. In one embodiment, the stent 120 is a covered stent. As shown in Fig. 1, the stent 120 includes a supporting framework 121 and a covered film 122 that is covered on the supporting framework 121. The supporting framework 121 has a hollow tubular cavity structure formed by weaving a metal wire or cutting a metal tube. The covered film 122 is covered in a circumferential direction of the supporting framework 121 to form a circumferentially closed tube cavity structure. The metal wire forms a loop-closed grid, such as a rhombic-shaped grid, a square grid, a triangular grid, and a circular grid.

When the stent 120 is assembled in the assembly instrument 110, the fixed handle 114 is operated with one hand, and the movable handle 115 is operated with the other hand, so that the outer sheath tube 113 slides towards the proximal end in the axial direction, that is, moves away from the sheath core tube 112 in the axial direction, so as to expose the sheath core tube 112. Then, the stent 120 is inserted over the sheath core tube 112 from one end with the guide head 111. Further, the stent 120 is crimped radially, so that the stent 120 is radially compressed and is fitted to an outer surface of the sheath core tube 112. Forming the loop-closed rhombic-shaped grid by the metal wire of the supporting framework 121 is taken as an example. When the stent 120 is radially compressed, the rhombic-shaped grid of the supporting framework 121 is still kept as a loop-closed grid having four end points. The free end of the assembly part 118 extends into the corresponding grid and is hooked to the stent 120 (the end surface of the free end of the assembly part 118 protrudes from the supporting framework 121, or is flush with the end surface of the metal wire of the supporting framework 121, or is lower than the end surface of the metal wire of the supporting framework 121, but is still hooked to the stent 120). The assembly part 118 can restrain the stent 120 from moving in the axial direction and the circumferential direction, which enables the stent 120 and the sheath core tube 112 to be relatively fixed, so as to avoid relative sliding between them. As shown in Fig. 1, the rhombic-shaped grid has four end points A, B, C, and D (point D is not shown in Fig. 1). When the stent 120 slides towards the guide head 111 relative to the sheath core tube 112, the assembly part 118 will contact point B on the rhombic-shaped grid, that is, the trend of the stent 120 for sliding towards the guide head 111 relative to the sheath core tube 112 is restrained by the assembly part 118. Similarly, the assembly part 118 can restrain the trend of the stent 120 for sliding away from the guide head 111. When the stent 120 tends to twist, it will contact point C or D, so that the assembly part 118 can restrain the trend of the stent 120 against twisting. Therefore, when the stent 120 is radially compressed and crimped on the sheath core tube 112, due to the limiting effect of the assembly part 118, the relative sliding between the stent 120 and the sheath core tube 112 is prevented. When the stent 120 and the assembly part 118 are kept in the hooked position, the stent 120 and the sheath core tube 112 are accommodated in the outer sheath tube 113 together to complete the assembly.

In one embodiment, the step of accommodating the stent 120 and the sheath core tube 112 into the outer sheath tube 113 together includes: keeping the outer sheath tube 113 stationary, and pushing the sheath core tube 112 and the stent 120 together into the inner cavity of the outer sheath tube 113 in the axial direction.

In another embodiment, the step of accommodating the stent 120 and the sheath core tube 112 into the outer sheath tube 113 together includes: keeping the sheath core tube 112 and the stent 120 stationary, pushing the outer sheath tube 113 in a direction close to the sheath core tube 112 and the stent 120 so that the sheath core tube 112 and the stent 120 are accommodated in the outer sheath tube 113.

Whether the sheath core tube 112 and the stent 120 are pushed together into the inner cavity of the outer sheath tube 113 in the axial direction or the outer sheath tube 113 is pushed in the direction close to the sheath core tube 112 and the stent 120, in the pushing process, the stent 120 and the sheath core tube 112 are kept relatively fixed all the time, which is conductive to avoiding the phenomenon of stacking. At the same time, it is conducive to avoiding twisting of the stent 120.

Therefore, the stent assembly system 100 is used to assemble the stent 120, which can avoid the stacking and twisting, thus improving the accuracy of assembly. Furthermore, the assembly efficiency and the assembly yield are improved. Meanwhile, no extra auxiliary fixtures are required in the assembling process, and the assembly method is simple.

As shown in Fig. 3, after the stent 120 enters the outer sheath tube 113, the assembly part 118 is still threaded in the rhombic-shaped grid of the stent 120, so that the limiting effect on the stent 120 can be still maintained. In other words, when the stent 120 is subjected to an axial or circumferential force from the sheath core tube 112, the assembly part 118 can effectively restrain the stent 120 to avoid its axial movement and circumferential rotation.

It can be understood that the stent 120 has a certain length. In order to ensure a better limiting effect, the number of the assembly part 118 is reasonably set according to the length of the stent 120. At the same time, the stent 120 has a tubular cavity structure, which is also used to ensure a better limiting effect. The orientations of the free ends of the plurality of assembly parts 118 can also be reasonably set. In the embodiment of Fig. 1, the number of assembly parts 118 is two, and the two assembly parts 118 are disposed at an interval. Furthermore, when the stent 120 is crimped on the sheath core tube 112, one assembly part 118 is located at the distal end of the stent 120, and the other assembly part 118 is located at the proximal end of the stent 120. The radial extending directions of the free ends of the two assembly parts 118 are opposite. The two assembly parts 118 can better limit the stent 120. Furthermore, since the two assembly parts 118 are respectively located at the distal end and the proximal end of the stent 120 in a spaced manner, the assembly parts 118 do not significantly increase the rigidity of the sheath core tube 112, so that the assembly instrument 110 can maintain its ability to pass through a bent lumen path.

In another embodiment, the extending directions of the free ends of the two assembly parts 118 may be the same. In this way, the limiting effect on the stent 120 can also be ensured.

The extending directions of the free ends of the assembly parts 118 are not parallel to the sheath core tube 112. In one embodiment, the assembly part 118 is perpendicular to a longitudinal center axis I-I of the sheath core tube 112. That is, the extending direction of the free end of the assembly part 118 is perpendicular to the longitudinal center axis I-I, as shown in Fig. 3.

In one embodiment, the assembly part 118 forms an acute angle with a longitudinal center axis I-I of the sheath core tube 112. That is, the extending direction of the free end of the assembly part 118 forms an acute angle with the longitudinal center axis I-I, and the assembly part 118 is inclined relative to the longitudinal center axis I-I, as shown in Fig. 4.

It should be noted that regardless of whether the assembly part 118 is perpendicular to the longitudinal center axis I-I or the assembly part 118 is inclined relative to the longitudinal center axis I-I, when there are a plurality of assembly parts 118, if the limiting effect is guaranteed to cause the stent 120 and the sheath core tube 112 to be kept relatively fixed, then is no limit on the way of disposing the plurality of assembly parts 118 on the sheath core tube 112. For example, the plurality of assembly parts 118 may be all located on the same side of the longitudinal center axis I-I; or, some assembly parts 118 are located on the same side of the longitudinal center axis I-I, and the other assembly parts 118 are located on the other side of the longitudinal center axis I-I. The plurality of assembly parts 118 located on the two sides of the longitudinal center axis I-I may or may not be radially opposite, that is, they may or may not be symmetrically disposed along the longitudinal center axis I-I. Alternatively, the free ends of the plurality of assembly parts 118 are alternately located on the two sides of the longitudinal center axis I-I of the sheath core tube 112.

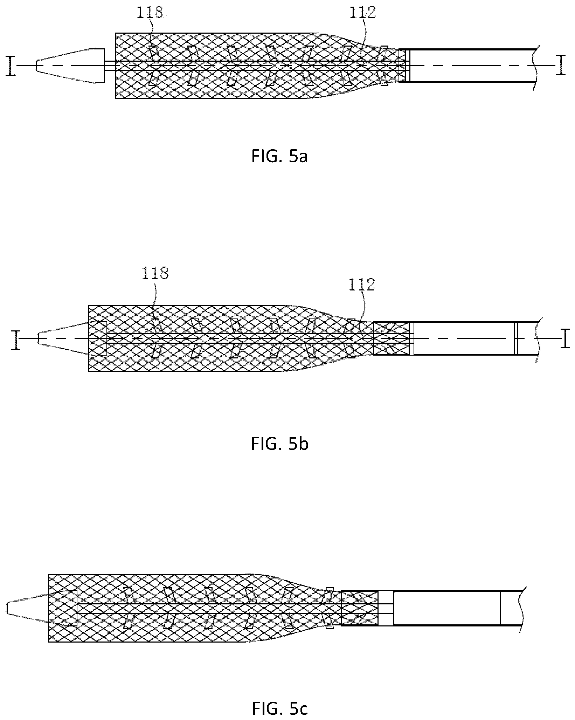

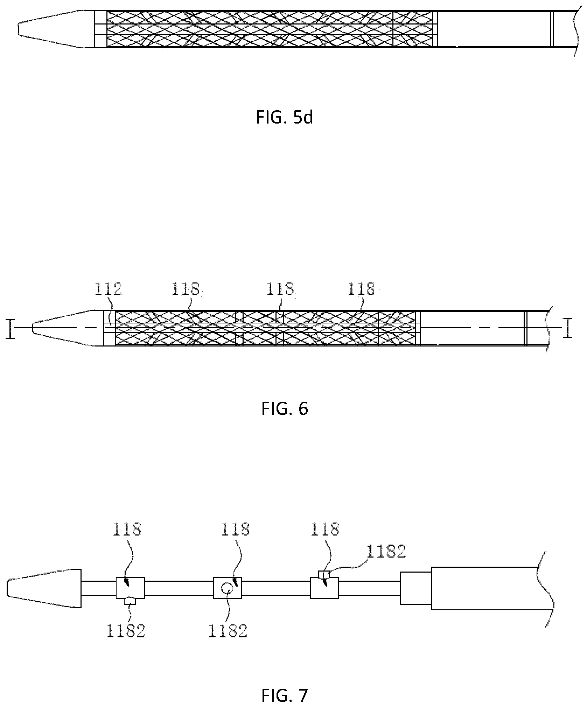

Referring to Fig. 5a, according to one embodiment, there are a plurality of assembly parts 118, and the plurality of assembly parts 118 all form an acute angle with the longitudinal center axis I-I of the sheath core tube 112. Every two of the plurality of assembly parts 118 form one group. By taking the longitudinal center axis I-I of the sheath core tube 112 as an axis of symmetry, each group is symmetrically arranged on the two sides of the longitudinal center axis I-I. The plurality of groups of assembly parts 118 are arranged at an interval in the axial direction of the sheath core tube 112. Furthermore, in the plurality of groups of assembly parts 118, some of them are inclined towards the distal end (which means that the free ends of the assembly parts 118 are closer to the distal end than the fixed ends), and some of them are inclined towards the proximal end (which means that the free ends of the assembly parts 118 are closer to the proximal end than the fixed ends). The plurality of groups of assembly parts 118 inclined towards the distal end are closer to the distal end than the plurality of groups of assembly parts 118 inclined towards the proximal end. In other words, from the distal end to the proximal end of the sheath core tube 112, the plurality of groups of assembly parts 118 inclined towards the distal end are arranged at an interval in the axial direction, and the plurality of groups of assembly parts 118 inclined towards the proximal end are arranged at an interval in the axial direction. Furthermore, the group of assembly parts 118 inclined towards the distal end, farthest away in the axial direction, of the guide head 111 are located at the distal ends of all the assembly parts 118 inclined towards the proximal end. The plurality of groups of assembly parts 118 inclined towards the distal end may or may not be arranged at equal intervals. The plurality of groups of assembly parts 118 inclined towards the proximal end may or may not be arranged at equal intervals. When the assembly parts 118 with different inclination directions are all arranged at equal intervals, the space between the plurality of groups of assembly parts 118 inclined towards the distal end may be equal to or unequal to the space between the plurality of groups of assembly parts 118 inclined towards the proximal end.

In order to cause the stent 120 and the sheath core tube 112 to move axially towards the outer sheath tube 113, a push force applied to the stent 120 and the sheath core tube 112 needs to turn the assembly part 118 from the state shown in Fig. 5b to the state shown in Fig. 5c, that is, the metal wire of the stent 120 can continue to press the assembly part 118 in Fig. 5c only when the orientation of the assembly part 118 changes, so that a space is generated between the assembly part 118 and the inner wall of the outer sheath tube 113, which causes the assembly part 118 to slide off from the space, and no limiting effect is achieved in the inner cavity of the outer sheath tube 113, which causes the stent 120 to move or twist. Compared to a vertical assembly part 118, the assembly part 118 in this orientation will not slide off until it is subjected to a relatively high acting force. Therefore, the assembly part 118 that is inclined away from the guide head 111 can effectively prevent the stent 120 from sliding towards the guide head 111 to cause the assembly part 118 to slide off.

After part of the stent 120 is assembled into the outer sheath tube 113, the stent 120 and the sheath core tube 112 is kept relatively fixed at the proximal end. The outer sheath tube 113 is continued to be pushed towards the guide head 111, so that the plurality of assembly parts 118 inclined towards the distal end can tilt more to comply with the outer sheath tube 113, so as to more conveniently enter the outer sheath tube 113.

By disposing the assembly parts 118 in different orientations, which are reasonably distributed on the sheath core tube 112, the limiting effect is provided for the stent 120, and assembly is facilitated.

In one embodiment, the plurality of groups of assembly parts 118 inclined towards the distal end and the plurality of groups of assembly parts 118 inclined towards the proximal end are disposed at unequal intervals. In a direction towards the distal end, the space of the plurality of groups of assembly parts 118 inclined towards the distal end is smaller. In a direction towards the proximal end, the space of the plurality of groups of assembly parts 118 inclined towards the proximal end is smaller. At the beginning of the assembly process, it is easiest for the stent 120 and the sheath core tube 112 to slide relative to each other. Therefore, the space of the plurality of groups of assembly parts 118 inclined towards the proximal end is smaller. More assembly parts 118 inclined towards the proximal end are disposed, so that at the beginning of the assembly process, the phenomenon that the stent 120 is stacked at or near the proximal end is avoided, and the part of the stent 120 that enters the outer sheath tube 113 is anchored, which is also conducive to keeping the stent 120 and the sheath core tube 112 to be relatively fixed to relieve or avoid a subsequent possible phenomenon of stacking in the middle or at the distal end.

As shown in Fig. 6, in one embodiment, there are a plurality of assembly parts 118. Every two of the plurality of assembly parts 118 form one group. By taking the longitudinal center axis I-I of the sheath core tube 112 as an axis of symmetry, each group is symmetrically arranged on the two sides of the longitudinal center axis I-I. The plurality of groups of assembly parts 118 are arranged at an interval in the axial direction of the sheath core tube 112. Furthermore, in the plurality of groups of assembly parts 118, some of them are inclined towards the distal end, some of them are inclined towards the proximal end, and some of them are perpendicular to the longitudinal center axis I-I of the sheath core tube 112. Furthermore, the assembly parts 118 (one group or multiple groups) inclined towards the distal end are located at the distal end; the assembly parts 118 (one group or multiple groups) inclined towards the proximal end are located at the proximal end; and the assembly parts 118 (one group or multiple groups) perpendicular to the longitudinal center axis I-I of the sheath core tube 112 are located in the middle. In this way, the accuracy of the assembly process can also be improved.

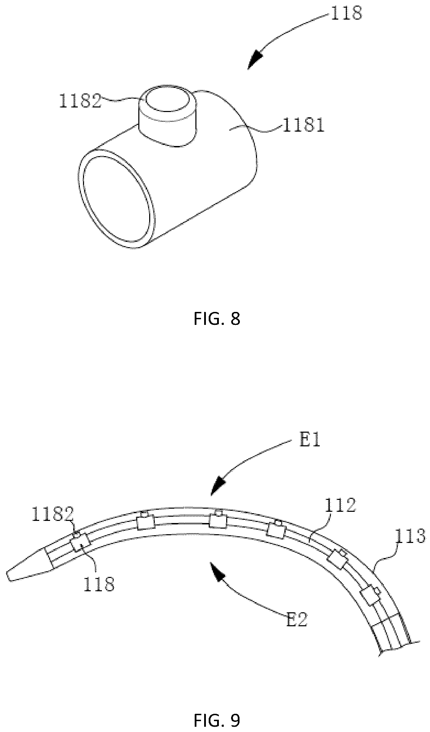

Referring together Fig. 7 and Fig. 8, in another embodiment, the assembly part 118 includes a base 1181 and a protrusion 1182 arranged on the base 1181. The base 1181 and the protrusion 1182 are of an integrated structure. Alternatively, the base 1181 and the protrusion 1182 are fixedly connected by welding, gluing, and the like. Materials of the base 1181 and the protrusion 1182 may be the same or different. The base 1181 is fixedly arranged on the outer wall of the sheath core tube 112, and the base 1181 is a fixed end of the assembly part 118. The protrusion 1182 extends from a connection part to the base 1181, and an end of the protrusion 1182 away from the base 1181 is a free end.

In one embodiment, as shown in Fig. 8, the base 1181 has a hollow structure, and the middle part of the base 1181 has an inner cavity. The base 1181 surrounds the sheath core tube 112 through the inner cavity. The protrusion 1182 is fixed on the outer wall of the base 1181. The assembly part 118 including the base 1181 is more easily and more reliably fixed to the sheath core tube 112. The base 1181 can lower the difficulty of the preparation process. For an assembly part 118 that does not include the base 1181, since the assembly part 118 has a relatively small size, the assembly part 118 and the sheath core tube 112 can be fixed by extreme precision. If a molding method is used to integrate the sheath core tube 112 and the assembly part 118, for stents 120 of different specifications or different types, the number of assembly parts 118 and the arrangement of the assembly parts 118 on the sheath core tube 112 may be different. Therefore, for different products, mold re-opening or frequent mold opening is possibly required. This inevitably increases the cost and the difficulty of the management and control.

Therefore, the assembly part 118 including the base 1181 and the protrusion 1182 lowers the difficulty of the preparation process, which is conducive to reducing the preparation cost. The space of the plurality of assembly parts 118 may be flexibly set according to a set requirement. Even if the base 1181 is added, the plurality of assembly parts 118 is still arranged on the sheath core tube 112 at intervals and do not significantly increase the rigidity of the sheath core tube 112. When reliability and fixation are satisfied, and the difficulty of the process is not significantly increased, the axial length of the base 1181 will possibly be small, so as to reduce the influence on the rigidity of the sheath core tube 112 to a minimum. Furthermore, for the outer sheath tube 113, clinically, it is generally hoped that the radial size of the outer sheath tube 113 is as small as possible. In order to not significantly occupy the internal space of the outer sheath tube 113, if the base 1181 has enough strength to cause the assembly part 118 to be reliably connected to the sheath core tube 112, the thickness of the base 1181 shall be as small as possible. In one embodiment, when the base 1181 is a hollow cylindrical, the ratio of the axial length of the base 1181 to the axial length of the protrusion 1182 is 1:1-1:25. The outer diameter of the sheath core tube 112 is d3. The outer diameter of the base 1181 is d4, d3+0.2mm≤d4≤d3+4mm.

In one embodiment, each assembly part 118 only includes one protrusion 1182. When a plurality of assembly parts 118 are arranged on the sheath core tube 112, the orientations of the protrusions 1182 may be the same or different. For example, as shown in Fig. 7, in the plurality of assembly parts 118, the orientations of the protrusions 1182 are different. Since the stent 120 has a tubular structure, the stent 120 surrounds the sheath core tube 112, and the orientations of the protrusions 1182 are different to achieve limitation at multiple parts in the circumferential direction, which is conducive to preventing the stent 120 from twisting relative to the sheath core tube 112. The plurality of assembly parts 118 may be regularly or irregularly arranged. For example, the plurality of assembly parts 118 can be regularly arranged on the sheath core tube 112 by being staggered by one phase. Being staggered by one phase means that in the diagram of the sheath core tube 112 along a cross section, the protrusions 1181 of the plurality of assembly parts 118 are circumferentially distributed, and a circumferential space of any two adjacent protrusions 1182 is equal.

In another embodiment, as shown in Fig. 9, a plurality of assembly parts 118 are arranged on the sheath core tube 112 at intervals. Each assembly part 118 only includes one protrusion 1182. The orientations of the protrusions 1182 of the plurality of assembly parts 118 are consistent. That is, the protrusions 1182 of the plurality of assembly parts 118 are located on the same side of the longitudinal center axis I-I (not shown in Fig. 9). Furthermore, the protrusions 1182 face a greater curvature side E1 of the outer sheath tube 113. When the stent assembly system 100 passes through a bent lumen path (such as a bent blood vessel), the outer sheath tube 113 and the stent 120 located in it will correspondingly bend to form the greater curvature side E1 and a lesser curvature side E2. On the lesser curvature side E2, the metal wire of the stent 120 will be gathered. On the greater curvature side E1, the metal wire becomes sparse, so that the risk of displacement increases. The protrusion 1182 of the assembly part 118 faces the greater curvature side E1, which can better limit the metal wire of the stent 120 located on the greater curvature side E1 to prevent the stent 120 from moving. Furthermore, the protrusion 1182 faces the greater curvature side E1, which is convenient for the stent assembly system 100 to pass through the bent lumen path.

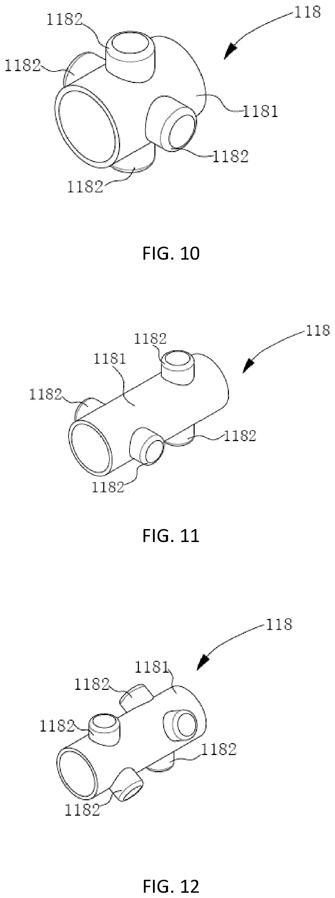

Referring to Fig. 10, Fig. 11, and Fig. 12, in other embodiments, each assembly part 118 includes a plurality of protrusions 1182. The plurality of protrusions 1182 are arranged at intervals in the circumferential direction of the base 1181. Alternatively, the plurality of protrusions 1182 are divided into two groups. The two groups of protrusions 1182 are arranged at an interval in the axial direction. Each group of protrusions 1182 is disposed at the interval in the circumferential direction. The plurality of protrusions 1182 are arranged at the interval in the circumferential direction of the base 1181, so as to maintained balanced limiting performance in the circumferential direction.

In one embodiment, the base 1181 of the assembly part 118 is a hollow cylinder. On the one hand, the machining is convenient. On the other hand, the connection between the assembly part 118 and the sheath core tube 112 is relatively reliable. It can be understood that in other embodiments, the shape of the base 1181 is not limited to the hollow cylindrical shape. Any shape that can fix the assembly part 118 on the sheath core tube 112 is available.

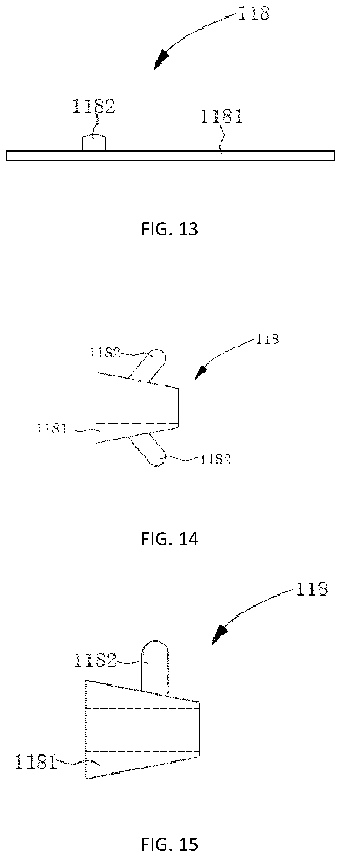

For example, as shown in Fig. 13, the base 1181 is an arc-shaped housing. The shape of the surface of the arc-shaped housing that extends away from the protrusions 1182 adapts to the arc-shaped surface of the sheath core tube 112. The arc-shaped housing is fitted to the outer wall of the sheath core tube 112, which is conducive to reliably fixing the assembly part 118 on the surface of the sheath core tube 112. Furthermore, the base 1181 does not completely wrap the sheath core tube 112 in the circumferential direction, which is convenient for avoiding a significant increase in the rigidity of the sheath core tube 112, so that the sheath core tube 112 is kept at a certain flexibility to facilitate passage through the bent lumen path. In one embodiment, the ratio of the axial length of the arc-shaped housing to the axial length of the protrusion 1182 is 1:1 to 1:25, and the thickness of the arc-shaped housing is 0.1 to 4 mm.

In another embodiment, referring to Fig. 14 and Fig. 15, the base 1181 is a hollow circular truncated cone, the outer contour of which is a circular truncated cone and the inner cavity of which is a cylinder. It should be noted that no matter how the shape of the base 1181 changes, the number of the protrusions 1182 of each assembly part 118 is not limited. The number may be one or multiple.

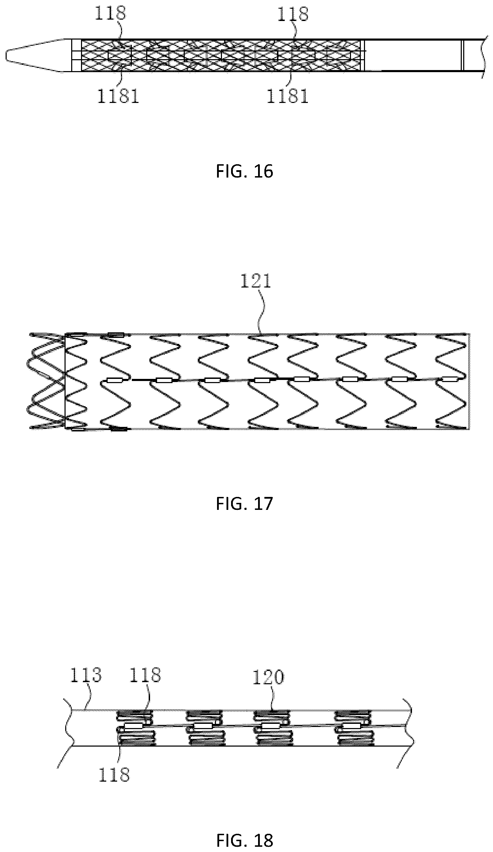

When the base 1181 is the hollow circular truncated cone, the orientation of the base 1181 is not limited. As shown in Fig. 16, in embodiment, the big ends of some bases 1181 are located at the proximal end, and the big ends of some bases 1181 are located at the distal end. The orientations of the corresponding protrusions 1182 are also different.

Regardless of the structure of the assembly part 118, such as a structure that only includes columnar protrusions or a structure that includes the base 1181 and the protrusion 1182, in one embodiment, the end surface of the free end of the assembly part 118 is a spherical surface, which is conducive to reducing scratches from the assembly part 118 to the inner wall of the outer sheath tube 113 and avoiding damage to the inner wall of the outer sheath tube 113.

The assembly part 118 is fixed on the outer wall of the sheath core tube 112 of the assembly instrument 110. In the assembly process, the free end of the assembly part 118 extends into the metal grid of the stent 120 and is hooked to the stent 120, so that the limiting effect is achieved, and relative sliding between the stent 120 and the sheath core tube 112 is avoided. The stent 120 and the sheath core tube 112 are kept relatively fixed, so that when the stent 120 and the sheath core tube 112 are together pushed into the outer sheath tube 113, the axial displacements of the stent 120 and the sheath core tube 112 can be kept consistent to avoid the phenomenon of stacking. Furthermore, after the stent 120 and the sheath core tube 112 are assembled in the outer sheath tube 113, the assembly part 118 still maintains the limiting effect on the stent 120 to prevent the stent 120 from twisting in the inner cavity of the outer sheath tube 113. Therefore, the accuracy of assembly of the assembly instrument 110 is high.

It should be noted that the metal wire of the stent 120 in the above embodiment is formed into the loop-closed grid. The assembly part 118 is caused to pass through the loop-closed grid to achieve the limiting effect. In another embodiment, regardless of whether the supporting framework 121 is prepared by weaving or cutting, the metal wire of the supporting framework 121 does not form a loop-closed grid. As shown in Fig. 17, the supporting framework 121 is a Z-shaped wave structure or a sine wave structure.

For the stent 120, the metal wire of which does not form the loop-closed grid, the above-mentioned assembly instrument 110 can also improve the accuracy of assembly. Fig. 18 is a schematic diagram illustrating that the stent 120 of the Z-shaped wave structure is assembled in the outer sheath tube 113. The assembly part 118 and the metal wire of the stent 120 are maintained in a hooked relationship. Specifically, the assembly part 118 is hooked to the lesser curvature side of a wave crest or a wave valley of a Z-shaped wave, so that the stent 120 can be limited in the axial direction and the circumferential direction. Thus, this arrangement avoids, during the assembly process, the phenomenon of stacking and prevents the stent 120 from twisting inside the outer sheath tube 113.

In one embodiment, regardless of whether the supporting framework 121 is prepared by weaving or cutting, and regardless of whether the metal of the supporting framework 121 forms the loop-closed grid or the non-loop-closed Z-shaped wave, sine wave, or other structures, the stent 120 does not include a covered film 122, i.e., the stent 120 is a bare stent. For a stent 120 that does not include the covered film 122, the limiting effect of the assembly part 118 is better. For a stent that includes the covered film 122, the assembly part 118, the outer sheath tube 113, and the covered film 112 are reasonably matched with each other, which can also achieve an effect of improving the accuracy of assembly.

The assembly part 118 shall be able to be hooked to the stent 120 and be able to be assembled into the outer sheath tube 113. At the same time, the stent 120 and the outer sheath tube 113 are not damaged.

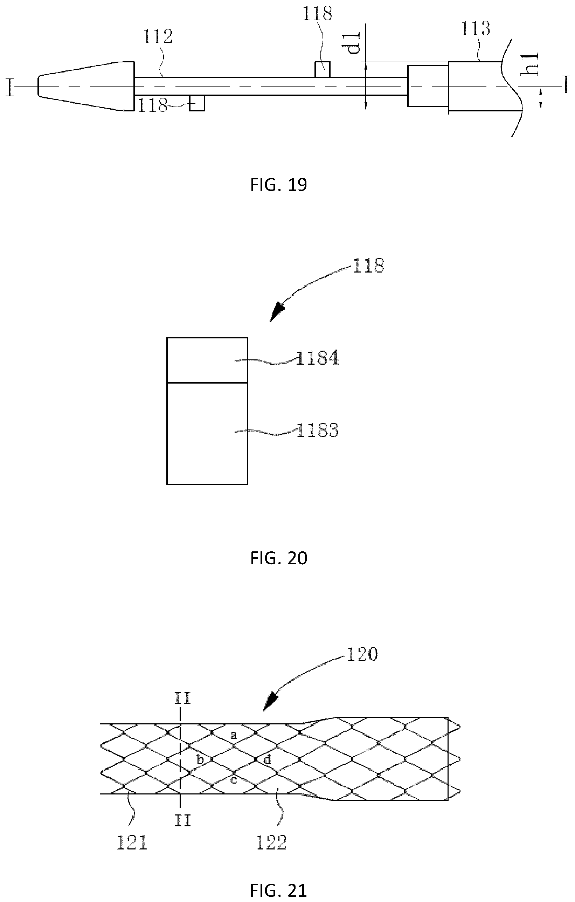

In the assembly process, after the stent 120 is crimped on the sheath core tube 112, the assembly part 118 extends into the rhombic-shaped grid of the stent 120, or the assembly part 118 is hooked to the wave crest or wave valley of the stent 120. Furthermore, after the stent 120 enters the outer sheath tube 113, the assembly part 118 will still extend into the rhombic-shaped grid, or still extend out of the wave crest or wave valley, so as to ensure the limiting effect and maintain the form of the stent 120 in the outer sheath tube 113. Therefore, the assembly part 118 shall have a certain height h1 (as shown in Fig. 19, the height is defined as a distance from the free end of the assembly part 118 to the longitudinal center axis I-I of the sheath core tube 112). When h1 is too small, it will be difficult to hook the assembly part 118 to the stent 120, so the limiting effect is hard to achieve. When h1 is too large, the protruding assembly part 118 may possibly scratch the inner wall of the outer sheath tube 113 during the process when the stent 120 and the sheath core tube 112 enter the outer sheath tube 113. On the one hand, assembly difficulty will be caused. On the other hand, the protruding assembly part 118 may possibly damage the inner wall of the sheath tube.

In one embodiment, the assembly part 118 has a certain elasticity, so that when the assembly part 118 can be properly extruded in the radial direction, h1 can be properly increased. In this way, the assembly part 118 can be reliably hooked to the stent 120 to effectively prevent relative sliding between the stent 120 and the sheath core tube 112. Furthermore, after the stent 120 is assembled into the outer sheath tube 113, the assembly part 118 can properly elastically resist against the inner wall of the outer sheath tube 113 to effectively prevent the stent 120 from moving. Meanwhile, when the stent 120 includes the covered film 122, and the assembly part 118 has a certain elasticity or lower degree of hardness, it is convenient to avoid damage to the covered film 122. Therefore, in one embodiment, the inner diameter of the outer sheath tube 113 is d1. When the hardness of the assembly part 118 is 25D-85D, 0.5d1<h1<0.8d1 to ensure that the free end of the assembly part 118 can extend out of the stent 120 and be reliably hooked to the stent 120, without damaging the covered film 122.

For the stent 120 that includes the covered film 122, the assembly part 118 will resist against the covered film 122 after extending out of the rhombic-shaped grid of the stent 120. When the height h1 of the assembly part 118 is too large, the free end of the assembly part 118 easily damages the covered film 122. Therefore, the height h1 of the assembly part 118 shall be reasonably selected in combination with the inner diameter d1 of the outer sheath tube 113, the wire diameter d2 of the metal wire of the stent 120 (when the supporting framework 121 is formed by cutting, d2 is the wall thickness of the supporting framework 121), and the thickness t of the covered film 122, so as to ensure a reliable limiting effect, prevent the stent 120 from twisting inside the outer sheath tube 113, and avoid damage to the covered film 122. In one embodiment, there is 0.35xd1-2xd2-t<h1<0.85xd1, so as to integrate the above three effects.

In one embodiment, for the stent 120 that includes the covered film 122, there is 0.35xd1-2xd2-t<h1<0.7xd1.

In one embodiment, for the stent 120 that does not include the covered film 122, h1 can be properly increased, so as to enhance the limiting effect on the stent 120. Particularly, for a bare stent formed by weaving, a weaving wire is relatively smooth, so that movement easily occurs in the assembly process. Therefore, h1 shall be properly increased, but it cannot be too large since an h1 that is too large may resist against the outer sheath tube 113. Meanwhile, when h1 is too small, the assembly part 118 can easily slide off from the metal wire, so that it is difficult to achieve the limiting effect. Therefore, in one embodiment, 0.4xd1-2xd2≤h1≤0.85xd1, so as to better achieve the limiting effect and the effect of preventing damage to the outer sheath tube 113. In one embodiment, 0.45Xd1-0.5mm ≤h1≤0.55Xd1+0.5mm, so as to better achieve the limiting effect and the effect of avoiding damage to the outer sheath tube 113.

Regardless of whether a metal wire is woven to form the loop-closed grid or the non-loop-closed Z wave, sine wave or other structures, or a metal tube is cut to form the loop-closed grid or the non-loop-closed Z wave, sine wave or other structures, the size of the loop-closed grid and the radius corresponding to the wave crest or wave valley of the non-loop-closed Z wave, sine wave or the like shall be within a certain range. When the stent 120 is compressed, the size of the grid and the size of the wave crest or wave valley will be correspondingly reduced. Therefore, the size of the cross-sectional area of the part of the assembly part 118 that is in contact with the grid, the wave crest or the wave valley shall be reasonable. On the one hand, the cross-sectional area shall not be too large, so that the assembly part 118 can be hooked to the stent 120 to provide the limiting effect. On the other hand, the cross-sectional area shall not be too small to ensure that the assembly part 118 has enough strength, to ensure the limiting effect, and to prevent the assembly part 118 from being broken from its base.

In one embodiment, the cross-sectional area (the area of a section parallel to the longitudinal center axis I-I of the sheath core tube 112) of the assembly part 118 is S1 (when the assembly part 118 is of a columnar structure, S1 is the sectional area of the columnar structure; when the assembly part 118 includes the base 1181 and the protrusion 1182, S1 is the sectional area of the protrusion 1182). When the metal wire of the stent 120 is formed into the loop-closed grid, in a natural spreading state, the grid area of the stent 120 is S2, there is 0.001 xS2 <S 1 <0.2xS2, so as to take into account the strength of the assembly part 118 and the limiting effect of the assembly part 118 on the stent 120.

In one embodiment, 0.002xS2≤S1≤0.08xS2.

In one embodiment, when the outer diameter of the outer sheath tube 113 is less than or equal to 14F, 0.03 mm²<S1<1.8 mm². In another embodiment, 0.07 mm²≤S1 ≤0.8 mm².

When the metal wire of the stent 120 is formed into the loop-closed structure, for example, the supporting framework 121 includes a plurality of waveform ring structures containing wave crests and wave valleys. In order to achieve the limiting effect, the assembly part 118 shall be hooked to the wave crests or wave valleys of the stent 120. That is, the assembly part 118 shall be threaded into a bent part of the metal wire. The radius of the bent part of the metal wire is R1. In one embodiment, 0.05xR1xR1x π≤S1≤R1xR1x π, so as to take into account the strength of the assembly part 118 and the limiting effect of the assembly part 118 on the stent 120.

In one embodiment, in order to take into account the strength of the assembly part 118 and to avoid damage to the outer sheath tube 113, when the assembly part 118 is of a columnar structure, as shown in Fig. 20, the assembly part 118 includes a rigid part 1183 and a flexible part 1184. The rigid part 1183 is the fixed end that is fixedly connected to the sheath core tube 112. One end of the flexible part 1184 is connected to the end of the rigid part 1183 away from the sheath core tube 112. The other end is the free end. When the stent 120 is crimped on the sheath core tube 112, the assembly part 118 is hooked to the stent 120. The free end of the flexible part 1184 resists against the covered film 122, which is conducive to avoiding damage to the covered film 122. In order to ensure that the overall assembly part 118 has enough strength to achieve the limiting effect in the assembling process, the hardness ratio of the rigid part 1183 to the flexible part 1184 is 0.3-0.9, and the length ratio is 1:5-5:1.

In one embodiment, when the assembly part 118 includes a base 1181 and a protrusion 1182, the protrusion 1182 includes a rigid part and a flexible part. The hardness ratio of the rigid part to the flexible part is 0.3-0.9, and the length ratio is 1:5-5:1.

In the existing research, in order to keep the stent 120 and the sheath core tube 112 relatively fixed, a friction element is usually arranged on a surface of the sheath core tube 112 to provide a surface friction force that prevents the stent 120 and the sheath core tube 112 from sliding relative to each other. Increasing the surface friction force can keep the stent 120 and the sheath core tube 112 relatively fixed to a certain extent, but the limiting effect of this method is limited, and it is difficult to achieve a reliable limiting effect. In the process of pushing to the inner cavity of the outer sheath tube 113, the stent 120 and the sheath core tube 112 will still be able to slide to a certain extent, which causes the phenomenon of stacking. In addition, by means of the method of limiting the surface friction force, the surface area of the friction element often needs to be increased, that is, it is better that the area of the surface of the sheath core tube 112 covered by the friction element is larger. In this way, the rigidity of the sheath core tube 112 will be increased, so that the flexibility of the assembly instrument 110 is reduced, and thereby increasing the difficulty for the assembly instrument 110 to pass through the bent lumen path. The assembly instrument 110 of the present disclosure does not significantly increase the rigidity of the sheath core tube 112 by disposing discontinuously distributed assembly parts 118 on the sheath core tube 112, thus not significantly reducing the flexibility of the assembly instrument 110. Moreover, the assembly part 118 is hooked to the stent 120 to provide the limiting effect. This limiting effect is more reliable than the friction effect. However, for the stent 120 that includes the covered film 122, since there is a possibility that the protruding assembly part 118 may perforate and damage the covered film 122, no one has set the assembly part 118 to limit the stent 120 in the assembly process. However, in the present disclosure, by reasonably setting the size of the assembly part 118 and the performance of the covered film 122, damage to the covered film 122 can be avoided while ensuring the limiting effect.

The material of the covered film 122 can be plastic, polyester or polyester. For example, the plastic may be polytetrafluoroethylene (PTFE). The polyester may be polyethylene terephthalate (PET) or polyurethane (PU), and the like. The reason why the covered film 122 can be perforated is that the covered film is subjected to an outward radial force from the assembly part 118. Two aspects can be considered to reduce the risk that the covered film 122 is perforated by the assembly part 118: on the one hand, the performance and size of the assembly part 118 itself, such as the hardness, the height h1, and the sectional area S1; on the other hand, the performance of the covered film 122 itself.

As for the size of the assembly part 118 itself, the height h1 needs to be as small as possible on the premise that the assembly part 118 can be reliably hooked to the stent 120, and the cross-sectional area S1 needs to be as large as possible on the premise that the assembly part 118 can be reliably hooked to the stent 120.

As for the performance of the cover film 122 itself, the covered film 122 should allow certain extension without being broken when it is subjected to a relatively large acting force from the assembly part 118. Therefore, the elongation of the covered film 122 should meet certain requirements. If the elongation of the covered film 122 is too small, the free end of the assembly part 118 may possibly resist against the covered film 122. Particularly for a stent 120 having a smaller metal grid, this possibility is higher, and folds of the covered film 122 formed completely by compression cannot ensure that the assembly part 118 extends into the grid of the metal wire. The surface of the covered film 122 is smoother. When the stent 120 is subjected to an external force, the assembly part 118 and the covered film 122 can more easily move relative to each other, and a good limiting effect cannot be achieved. However, when the elongation of the covered film 122 is too large, under the action of the assembly part 118, it is easy for the covered film 122 to protrude, so that the partial thickness of the covered film 122 is reduced, which forms a thin part. This will increase the risk that after the stent 120 is released, due to the action of blood pressure, the thin part is broken or blood permeates through the thin part.

Therefore, in one implementation mode, 95% ≤ elongation ≤ 300%.

A test method for the elongation is as follows: a covered film material is cut into a test sample having a length greater than 90 mm and a width equal to 15 mm; the test sample is fixed on clamps of a tensile machine; the distance between two clamps is 50 mm; the covered film is slowly stretched until the sample is broken. A maximum force F in this process and a stretching length L1 of the covered film at the maximum force are recorded, so that there is elongation=(L1-50)/50^{∗}100%.

Meanwhile, the breaking strength of the covered film 122 will also generate a certain influence. When the breaking strength of the covered film 122 is too low, it is very easy for the assembly part 118 to penetrate the covered film 122, which causes the stent 120 to fail. However, the covered film 122 with higher breaking strength generally has smaller porosity or greater thickness. The reduction of the porosity will affect the endothelium climbing effect, and the increase of the thickness of the covered film 122 will reduce the flexibility of the stent 120.

Therefore, in one embodiment, 0.5 N/mm ≤breaking strength≤15.0 N/mm. In another embodiment, 1.0 N/mm≤ breaking strength ≤9.5 N/mm.

The breaking strength of the covered film material is obtained according to the above-mentioned test method for the elongation, breaking strength= F/15 mm.

Furthermore, the thickness t of the covered film 122 and the height h1 of the assembly part 118 shall satisfy a certain relationship. When t is larger, and h1 is smaller, it is difficult for the assembly part 118 to push up the covered film 122, which cannot ensure that the assembly part 118 is reliably hooked to the stent 120. When t is smaller, the covered film 122 is thinner. When h1 is larger, the perforation risk for the covered film 122 is higher. Therefore, in one embodiment, 0.010≤ t/h1 ≤ 0.500. In another embodiment, 0.013 ≤ t/h1 ≤0.250.

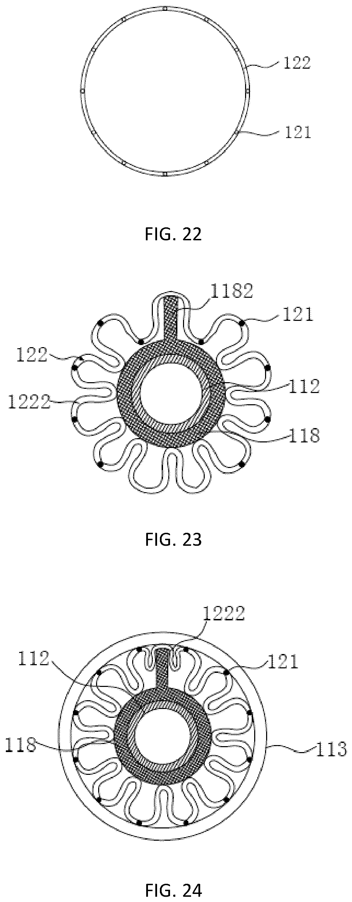

The form of the covered film itself 122 also has a certain influence on avoiding damage to the assembly part 118. Referring to Fig. 21 and Fig. 22, in a natural state, the sectional form of the stent 120 is approximately circular. When the stent 120 is radially crimped onto the sheath core tube 112, as shown in Fig. 23 and Fig. 24, the covered film 122 between adjacent metal wires is basically in an arc-shaped open state to form a plurality of folds 1222. The folds 1222 provide a certain space for the assembly part 118. The free end of the assembly part 118 extends into the fold 1222, which prevents the covered film 122 from being perforated by the assembly part 118 when the stent 120 is located inside the outer sheath tube 113 during the assembly process. The stent 120 is compressed due to the radial compression force. The radial compression force is actually applied to the supporting framework 121 of the stent 120. Under the action of the radial compression force, the supporting framework 121 will be extruded towards a direction of being secured to the outer surface of the sheath core tube 112. In this state, if the covered film 122 located between the metal wires of the supporting framework 121 is in a tightened state, the risk that the covered film 122 is perforated by the radially protruding assembly part 118 is higher. Thus, in the compressed state, the covered film 122 forms proper folds 1222, which is conducive to reducing the risk that the covered film 122 is perforated by the assembly part 118.

An appropriate compression rate can ensure that the covered film 122 between the adjacent metal wires is in a folded state. When the stent 120 is compressed, the distance between the adjacent metal wires is shortened. The covered film 122 forms the fold 1222 in the compression process. In the natural state, the diameter of the stent 120 is set as D1, and the inner diameter of the outer sheath tube 113 is set as d1. It is defined that the compression rate of the stent 120 is (D1-d1)/D1. A larger compression rate indicates that the stent 120 is compressed to a larger extent. If the compression rate is smaller, from the natural state to the compressed state for assembly, the space of the adjacent metal wires in any grid of the stent 120 changes less; the fold formed by the covered film 120 is smaller; and the covered film is more easily perforated by the assembly part 118. In one embodiment, referring back to Fig. 21, the supporting framework 121 is composed of a plurality of rhombic-shaped grids. Any rhombic-shaped grid has four intersections: a, b, c, and d. Point b and point d are distributed at an interval in the axial direction, and point a and point c are distributed at an interval in the radial direction. It can be understood that when the stent 120 is in the compressed state, the degree of fold of the covered film 122 closer to the intersection is smaller, and the degree of fold of the covered film 122 farther from the intersection is larger. The degree of fold in the center of the grid is the largest. Therefore, the covered film 122 closer to the intersection is more easily perforated by the assembly part 118. Therefore, if the compression rate is smaller, the risk that the part of the covered film 120 in the grid of the stent close to the intersection is perforated by the assembly part 118 significantly increases. It can be understood that the assembly part 118 should protrude from the center of the rhombic-shaped grid, but the assembly part 118 may possibly deflect to a position close to the intersection due to the accuracy of a production process and under the action of an axial pushing force during the assembly, and the covered film 122 is actually broken near the intersection, particularly point b and point d.

However, if the compression rate is too large, when the stent 120 and the sheath core tube 112 is pushed into the outer sheath tube 113, there is an insufficient gap to accommodate the assembly part 118. By forcible pushing, even if the sheathing action succeeds, a partial release force for the outer sheath tube 113 will be high, which will make it difficult to release the stent 120.

Therefore, in one embodiment, 40% ≤ compression rate ≤ 85%. Within this compression rate range, the safety of the assembly process, the convenience of delivery, and the convenience of release are taken into account.

When the covered film 122 is of a double-layer structure, including an inner-layer film and an outer-layer film, and the supporting framework 121 is located between the inner-layer film and the outer-layer film, for the supporting framework 121 formed by weaving, the grid has four intersections a, b, c, and d. When the assembly part 118 is located near the intersections a, b, c, and d and is subjected to an acting force towards the intersections, the intersected metal wires tend to slide relative to each other. The metal wires are stressed near the intersections and move towards each other, which will separate the inner-layer film from the outer-layer film. In a severe case, the stent 120 may fail.

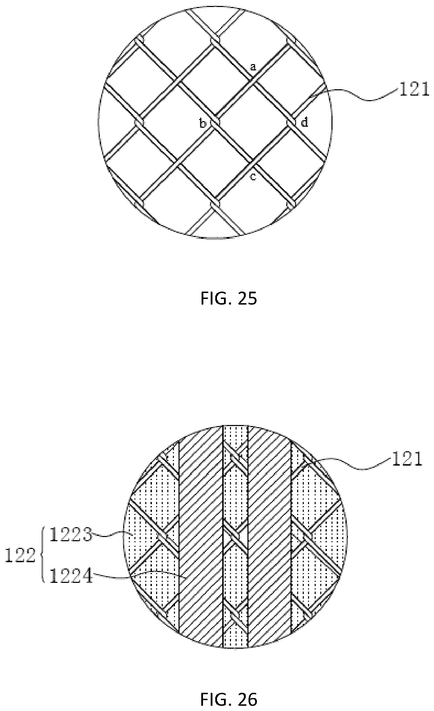

In one embodiment, as shown in Fig. 21, the supporting framework 121 of the stent 120 is woven in an intersecting and mutual hooking manner. This intersecting and mutual hooking design can effectively prevent the separation of the inner-layer film from the outer-layer film of the covered film 122 caused by the fact that the metal wires near the intersections slide relative to each other due to the action of the assembly part 118. In the embodiment of Fig. 21, each intersection has mutual hooking design. In another embodiment shown in Fig. 25, in the metal grid of the supporting framework 121, only point b and point d in the axial direction are mutually hooked, and point a and point c are intersected. In other words, they are overlapped with each other, instead of being mutually hooked. Because the stent 120 is not subjected to an extra circumferential outer force during assembly, the risk of circumferential movement is lower than that of axial movement. Therefore, the mutually hooked intersections are formed in the axial direction only, which can preferentially ensure that the axial movement will not cause the covered film 122 to be broken at the intersections. Furthermore, the overall number of intersections of the stent 120 can be reduced, so that the stent 120 has a certain flexibility.

Due to the intersecting and mutual hooking design, the metal wires are fixedly connected at the intersections, which can restrain the movement of the metal wires at the intersections to a certain extent. However, in general, when the covered film 122 is of a single-layer structure, the covered film 122 has a one-piece structure. When the covered film 122 is of the double-layer structure including the inner-layer film and the outer-layer film, the inner-layer film and the outer-layer film are respectively of one-piece structures. The mutually hooked metal wires can rotate around hinges within a certain range. This will also cause the covered film 122 to be separated or broken. Therefore, in embodiment, the metal wires of the stent 120 are mutually hooked only at the axial intersections. Furthermore, as shown in Fig. 26, the covered film 122 includes an inner-layer film 1223 and an outer-layer film 1224. The inner-layer film 1223 has a barrel-shaped one-piece structure, and the inner-layer film 1223 is wrapped by the supporting framework 121 in the circumferential direction to form a circumferentially closed tube cavity structure. The outer-layer film 1224 has a strip-type structure. The strip-type outer-layer film 1224 is wound on the metal wires and the outer surface of the inner-layer film 1223, and the metal wires located on the mutually hooked intersections are not covered by the outer-layer film 1224; that is, no outer-layer film 1224 is disposed at the intersections. In this manner, when the assembly part 118 resists against the intersections of the covered film 122 or a position close to the intersections, it only acts on the inner-layer film 1223 without applying an acting force on the outer-layer film 1224. Therefore, the separation of the inner-layer film 1223 from the outer-layer film 1224 can be avoided.

By the above-mentioned cooperation method of the inner-layer film 1223 and the outer-layer film 1224, the supporting framework 121 and the covered film 122 are reliably fitted, and it is favorable for avoiding the movement of the inner-layer film 1223 and the outer-layer film 1224 under the action of the assembly part 118.

In one embodiment, the outer-layer film 1224 is spirally wound on the metal wire and the outer surface of the inner-layer film 1223 to prevent the intersections from being covered. It can be understood that the arrangement of the outer film 1224 is not limited to being spirally wound on the metal wire and the outer surface of the inner-layer film 1223. Any arrangement that can avoid the outer-layer film 1224 from covering the hooked positions of the metal wires is acceptable.

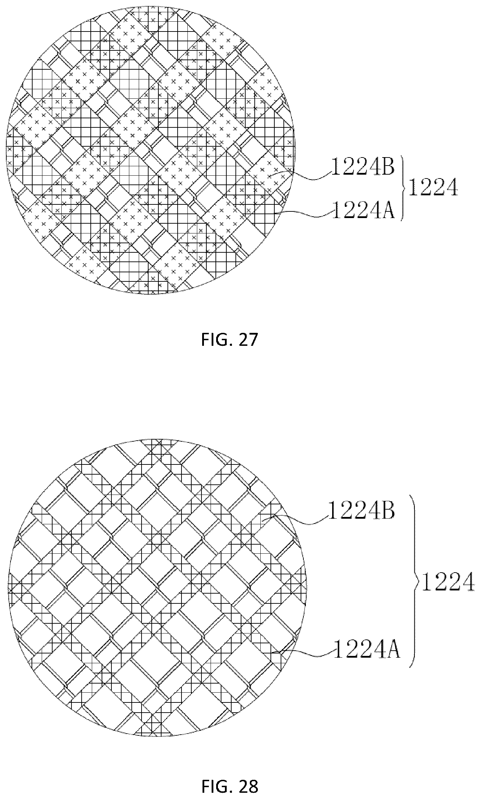

Referring to Fig. 27, in one embodiment, the outer-layer film 1224 includes two parts, i.e., including a plurality of first strip-type films 1224A and a plurality of second strip-type films 1224B. The plurality of first strip-type films 1224A cover the metal wire and the outer surface of the inner-layer film 1223 (the inner-layer film 1223 is omitted in Fig. 27) in parallel at an interval. The plurality of second strip-type films 1224B cover the metal wire and the outer surface of the inner-layer film 1223 in parallel at another interval. Furthermore, the first strip-type films 1224A and the second strip-type films 1224B are intersected, and both the first strip-type films 1224A and the second strip-type films 1224B cover the mutually hooked intersections of the metal wires. In this way, the metal wires are more reliably fitted to the outer-layer film 1224.

Referring together to Fig. 27 and Fig. 28, when it is ensured that the metal wire is reliably fitted to the outer-layer film 1224 to avoid the mutually hooked intersections of the metal from being covered, the widths of the first strip-type films 1224A and the second strip-type films 1224B can be reasonably set.

It should be noted that in other embodiments, the outer-layer film 1224 includes a first strip-type film 1224A and a second strip-type film 1224B. The first strip-type film 1224A and the second strip-type film 1224B are both of continuous structures and are wound. Furthermore, the first strip-type film 1224A and the second strip-type film 1224B are intersected. Both the first strip-type film 1224A and the second strip-type film 1224B cover the mutually hooked intersections of the metal wires. Reasonably setting the performance of the covered film 122 and the assembly part 118 will improve the accuracy of assembly of the stent assembly system 100. Meanwhile, setting the assembly part 118 to improve the accuracy of assembly is also applicable to the stent 120 that includes the covered film 122.

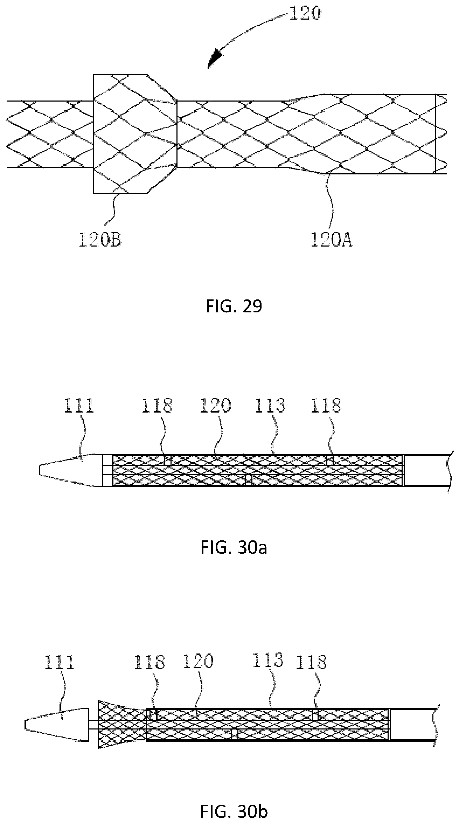

The assembly instrument 110 is not only applicable to a single-layer stent, but also applicable to a double-layer stent. Referring to the embodiment of Fig. 29, the stent 120 has a partial double-layer structure. The stent 120 includes a first tube body 120A and a second tube body 120B. The first tube body 120A has a tubular cavity structure with openings at two ends. The second tube body 120B surrounds the first tube body 120A, and at least one end of the second tube body 120B is fixedly connected to a peripheral surface of the first tube body 120A. Furthermore, the peripheral surface of the first tube body 120A is partially covered by the second tube body 120B. Both of the first tube body 120A and the second tube body 120B can be covered stents or bare stents. A plurality of assembly parts 118 are arranged on the sheath core tube 112 at intervals. Furthermore, the height h1 of the assembly 118 simultaneously opposite to the first tube body 120A and the second tube body 120B in the radial direction is less than that of the assembly part 118 opposite to the first tube body 120A in the radial direction, but it still needs to ensure that the assembly part 118 has a sufficient height h1 to be hooked to the stent 120 to achieve a limiting effect. This is because the inner cavity of the outer sheath tube 113 is an equal-diameter inner cavity. When the stent 120 is assembled in the outer sheath tube 113, since the stent 120 has a partial double-layer structure, the second tube body 120B inevitably occupies a certain space of the inner cavity of the outer sheath tube 113. When the height h1 of the assembly part 118 simultaneously opposite to the first tube body 120A and the second tube body 120B in the radial direction is too large, there is risk of damage to the stent 120 and/or the outer sheath tube 113.

In one embodiment, when both of the first tube body 120A and the second tube body 120B are covered stents, the height h1 of the assembly part 118 simultaneously opposite to the first tube body 120A and the second tube body 120B in the radial direction satisfies this relationship: 0.4xd1-2xd2-t<h1<0.8xd1, so that the free end of the assembly part 118 protrudes from the metal wires of the first tube body 120A, without damaging the covered film.

In one embodiment, when the first tube body 120A and the second tube body 120B are both bare stents, the height h1 of the assembly part 118 simultaneously opposite to the first tube body 120A and the second tube body 120B in the radial direction satisfies this relationship: 0.35xd1-2xd2-t<h1<0.75xd1, so that the free end of the assembly part 118 protrudes from the metal wires of the first tube body 120A, without damaging the inner wall of the outer sheath tube 113.

In one embodiment, when the second tube body 120B does not cover two ends of the first tube body 120A, in the embdiment as shown in Fig. 29, the assembly part 118 simultaneously opposite to the first tube body 120A and the second tube body 120B in the radial direction can be omitted. The assembly part 118 is arranged at the proximal end and the distal end o the first tube body 120, which can also achieve limitation to the stent 120.

The stent assembly system 100 improves the accuracy of assembly from the source, which can avoid the influence of low accuracy of assembly on the release accuracy in the operation. A release process of the stent 120 is described below in combination with Fig. 30a to Fig. 30d.

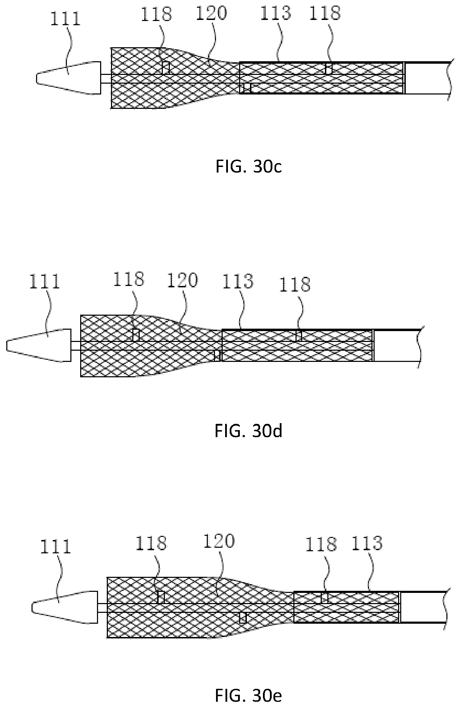

After the assembly instrument 110 delivers the stent 120 to a lesion site, the stent 120 is still located in the inner cavity of the outer sheath tube 113, as shown in Fig. 30a, and the assembly part 118 is kept in hooked connection to the stent 120. By keeping the sheath core tube 112 stationary, the outer sheath tube 113 moves axially away from the guide head 111. The stent 120 is in contact with the inner wall of the outer sheath tube 113. Under the action of the friction force of the outer sheath tube 113, the stent 120 tends to move away from the guide head 111 together with the outer sheath tube 113. If the stent 120 has obviously moved away from the guide head 111, the part of the stent 120 that is in the inner cavity of the outer sheath tube 113 will be extruded. In a severe case, it may be stacked, which will reduce the accuracy of the release. For example, the position of the stent 120 may be deviated or the form of the stent 120 may be twisted. Furthermore, the release position will be deviated. As shown in Fig. 30b, due to the arrangement of the assembly part 118, the assembly part 118 is kept in hooked connection with the stent 120. As the outer sheath tube 113 moves, the stent 120 can resist the friction of the outer sheath tube 113, so that the stent 120 is kept fixed with respect to the sheath core tube 112. Referring together to Fig. 30a and Fig. 30e, the plurality of assembly parts 118 are arranged at intervals in the axial direction of the sheath core tube 112. As the outer sheath tube 113 gradually moves away from the guide head 111, the stent 120 is gradually released. However, the part located in the inner cavity of the outer sheath tube 113 is at least partially hooked to the assembly part 118, which can effectively prevent the movement of the stent 120, thus improving the release accuracy. Due to the restoration of the stent 120 itself, when the restriction from the outer sheath tube 113 to the stent 120 disappears, the stent 120 automatically pops up and is fitted to the inner wall of a tissue, so as to complete the release.

In order to ensure that the stent 120 is always limited by the assembly part 118 during the whole process of release and is kept fixed with respect to the sheath core tube 112 in the whole process, in one embodiment, the assembly part 118 farthest away from the guide head 111 in the axial direction is hooked to the metal grid of the stent 120 farthest away from the guide head 111 in the axial direction or hooked to the wave crest or wave valley, that is, at least one assembly part 118 is located at the most proximal end of the stent 120.

The stent 120 is gradually released. During the release process, when the ratio of the axial length of the released part of the stent 120 to the axial length of the part of the stent 120 located in the outer sheath tube 113 is larger, since the stent 120 will automatically pop up, the most distal end (i.e., the part that is released at the very beginning) of the stent 120 is in at least some contact with the inner wall of the tissue to achieve a certain limiting effect. Therefore, even if the assembly part 118 that is farthest away from the guide head 111 in the axial direction is away from the most proximal end of the stent 120 to a certain extent, it can be also ensured that during the whole release process, relative sliding between the stent 120 and the sheath core tube 112 is avoided.

In one embodiment, when the stent 120 is radially crimped on the sheath core tube 112 (in a state that the stent 120 is hooked to all the assembly parts 118), the ratio of the axial distance between the assembly part 118 located at the most proximal end and the proximal end surface of the stent 120 to the axial length of the stent 120 is not greater than 1/3.

In one embodiment, the space of the plurality of assembly parts 118 close to the distal end is smaller, which is favorable for the release. This is because, during the release process, the release force at the very beginning of the release is the highest, which is most likely to cause the distal end of the stent 120 to move so as to lead to stacking, resulting in failure during release. Therefore, more assembly parts 118 are arranged in a direction close to the distal end, so that the stent 12 can be better restrained from moving to avoid the failure during release, or to reduce the difficulty of release.

In one embodiment, when the stent 120 is radially crimped on the sheath core tube 112 (in a state that the stent 120 is hooked to all the assembly parts 118), the ratio of the axial distance between the assembly part 118 located at the most distal end and the distal end surface of the stent 120 to the axial length of the stent 120 is not greater than 1/3, which is favorable for avoiding stacking at the proximal end in the assembly process.

The traditional stent delivery system often cannot withdraw the stent after the stent is partially released. Therefore, once the stent is twisted after the release, or cannot be continued to be released after one part has been released, or other problems occur, there is no solution. In this situation, an implant can only be taken out through a surgical operation. This increases the clinical risk to a patient. Or, when a release position is not accurate, it is also hard to adjust the release position through withdrawal, which will affect the treatment effect. The stent assembly system 100 of the present disclosure can achieve withdrawal, thus avoiding the above-mentioned problems.

It can be understood that the order of withdrawal of the stent 120 and the order of release are opposite. First, referring to Fig. 30e, when an abnormality is found after one part of the stent 120 is released and the stent needs to be withdrawn, the sheath core tube 112 is kept stationary to cause the outer sheath tube 113 to move towards the guide head 111. During the process that the outer sheath tube 113 moves towards the guide head 111, the inner wall of the outer sheath tube 113 will impart friction onto the outer wall of the stent 120, so that a pushing force towards the guide head 111 is applied to the stent 120. Therefore, the stent 120 tends to move together with the outer sheath tube 113; that is, it tends to move with respect to the sheath core tube 112. However, since the part of the stent 120 that is located in the inner cavity of the outer sheath tube 113 is still kept in hooked connection with the assembly part 118, the assembly part 118 will apply a certain acting force to the stent 120 to resist the movement, thus preventing the stent 120 from moving. Therefore, the withdrawal of the stent 120 can be achieved by means of pushing the outer sheath tube 113 towards the guide head 111. The state changes in the withdrawal process are shown in sequence in Fig. 30e, Fig. 30d, Fig. 30c, Fig. 30b, and Fig. 30a. In the process that the outer sheath tube 113 moves forward towards the guide head 111, the grid of the part of the stent 120 located outside the outer sheath tube 113 gradually gets close to the assembly part 118 until the assembly part 118 is threaded into the grid. The outer sheath tube 113 continues to be pushed towards the guide head 111. The assembly part 118 and the stent 120 are together received into the outer sheath tube 113 to achieve the withdrawal of the stent 120. Since a traditional stent delivery system is not provided with an assembly part, if withdrawal is attempted after one part of the stent 120 is released, the stent 120 will move with the outer sheath tube 113 during the operation, making it difficult to achieve the withdrawal.

By means of the design of the structure and number of the assembly parts 118, in the assembly process, the assembly parts 118 can provide a certain reliable movement-resisting force to the stent 200, so that the stent will not be pulled apart to achieve the withdrawal of the stent 120. Furthermore, as mentioned above, by means of the arrangement of the covered film for the assembly parts 118 and the stent 120, during the withdrawal process, the assembly parts 118 will not perforate the covered film 122 or damage the covered film 122. Therefore, the position can be adjusted by withdrawing the stent 120, so as to continue to release the stent 120.

Therefore, since the assembly part 118 is reasonably set, the stent assembly system 100 will improve the accuracy from the assembling source. Furthermore, the accuracy can be improved during the release process, which is favorable for successfully performing the interventional operation and ensuring the treatment effect. Moreover, in the case of an accident, withdrawal can also be achieved, so that it is safe and reliable.

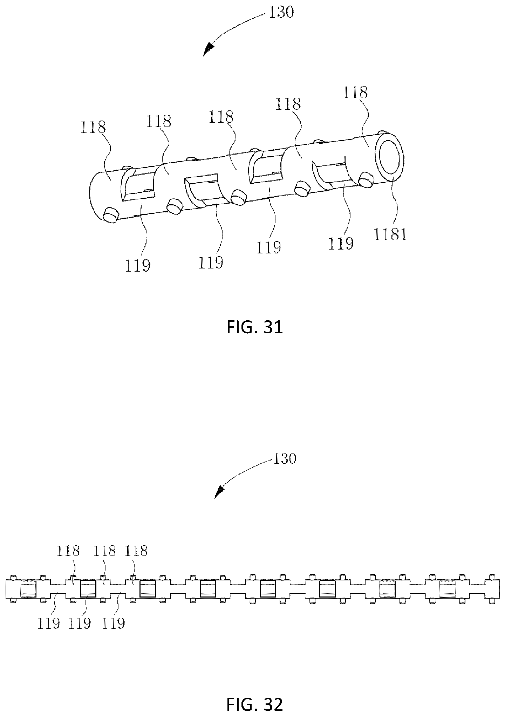

It should be noted that when the assembly part 118 can be reliably hooked to the stent 120, and the assembly part 118 does not damage the covered film 122 and/or the outer sheath tube 113, the assembly part 118 is not limited to the above-mentioned specific structure and may be embodied in other structures. For example, referring to Fig. 31, a plurality of assembly parts 118 are connected through connecting members 119 to form an assembled component 130. During assembly, the assembled component 130 is directly mounted on the sheath core tube 112.

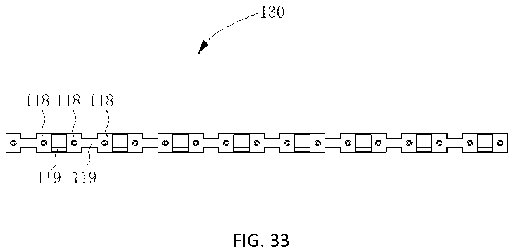

Referring to Fig. 32 and Fig. 33, the connecting members 119 are approximately rod-like, which can be flat rods or arc-shaped rods. Any two adjacent assembly parts 118 can be connected through at least one connecting member 119. Two ends of each connecting member 119 are respectively connected to the bases 1182 of two adjacent assembly parts 118. The number of the assembly parts 118 of each assembled component 130 can be reasonably set according to the length of the stent 120. The number of the assembled components 130 on each assembly instrument 110 can be reasonably set by considering the length of the stent 120 and the number of the assembly parts 118 of each assembled component 130. It is more convenient to form the assembled component 130 by connecting, via the connecting members 119, the plurality of assembly parts 118. Since the size of each assembly part 118 is very small, preparing a single assembly part 118 has higher requirements for a mold, so as to meet precision requirements. The size of the assembled component 130 is relatively large, which has lower requirements for the mold, and the assembled component can be injection-molded at one time.

The number and arrangement positions of the connecting members 119 between two adjacent assembly parts 118 may be the same or different. As shown in Fig. 31, in one embodiment, two adjacent assembly parts 118 are connected by two connecting members 119 disposed at an interval in the circumferential direction of the sheath core tube 112. In combination with Fig. 32 and Fig. 33, for the non-end assembly parts 118, a plane formed by two connecting members 119 at the distal end is perpendicular to a plane formed by two connecting members 119 at the proximal end. Setting the connecting members 119 in this way is beneficial to ensure that the flexibility of the assembly instrument 110 in any angular direction is consistent, and is beneficial to pass through a complex bent path.

The technical features of the embodiments described above can be arbitrarily combined. In order to make the description concise, all possible combinations of various technical features in the above embodiments are not completely described. However, the combinations of these technical features should be considered as the scope described in the present specification as long as there is no contradiction in them.

The above-mentioned embodiments only express several embodiments of the present disclosure, and their descriptions are more specific and detailed, but they cannot be understood as limiting the patent scope of the present disclosure. It should be noted that those of ordinary skill in the art can further make various transformations and improvements without departing from the concept of the disclosure, and these transformations and improvements all fall within the protection scope of the present disclosure. Therefore, the protection scope of the patent of the present disclosure shall be subject to the appended claims.

## Claims

1. A stent assembly system, comprising a stent and an assembly instrument, wherein the assembly instrument is used to assemble and deliver the stent; the assembly instrument comprises a sheath core tube, an outer sheath tube and an assembly part; the outer sheath tube slidably surrounds the sheath core tube in an axial direction, and an accommodating cavity for accommodating the stent is formed between the inner wall of the outer sheath tube and the outer wall of the sheath core tube; the assembly part has a fixed end and a free end opposite to the fixed end; the fixed end is connected to the sheath core tube; and when the stent is radially crimped on the sheath core tube, the free end is hooked to the stent to limit the stent.

2. The stent assembly system of claim 1, comprising a plurality of assembly parts; the plurality of assembly parts are arranged on the sheath core tube at intervals; when the stent is radially crimped on the sheath core tube, wherein the ratio of an axial distance between the assembly part located at the most proximal end and the proximal end surface of the stent to the axial length of the stent is not greater than 1/3.

3. The stent assembly system of claim 1, wherein the free end of the assembly part has a spherical structure.

4. The stent assembly system of claim 1, wherein an inner diameter of the outer sheath tube is d1; a height of the assembly part is h1; and when the hardness of the assembly part is 25D-85D, there is the relationship 0.5d1<h1<0.8d1.

5. The stent assembly system of claim 1, wherein the stent comprises a supporting framework and a covered film that surrounds the supporting framework; the supporting framework has a grid structure formed by weaving metal wires; at least some metal wires are intersected and mutually hooked to form a plurality of intersections; the covered film comprises an inner-layer film and an outer-layer film; the inner-layer film is arranged in the supporting framework; the outer-layer film is attached to an outer surface of the supporting framework and an outer surface of the inner-layer film; and the plurality of intersections are not covered by the outer-layer film.

6. The stent assembly system of claim 5, wherein at least some metal wires are intersected and mutually hooked to form a plurality of intersections that are spaced apart from each other in the axial direction.

7. A stent assembly method, **characterized by** the following steps:
sliding an outer sheath tube towards a proximal end in an axial direction until the outer sheath tube is exposed from the sheath core tube;
surrounding a stent on a sheath core tube;
radially crimping the stent so that the stent is radially compressed and attached to an outer surface of the sheath core tube and the assembly part is hooked to the stent; and
wherein in the state that the assembly part and the stent are hooked, accommodating the sheath core tube and the stent together into the outer sheath tube.

8. The stent assembly method of claim 7, wherein in the state that the assembly part and the stent are hooked, the step of accommodating the sheath core tube and the stent together into the outer sheath tube comprises: keeping the outer sheath tube stationary, and pushing the sheath core tube and the stent together into the outer sheath tube in the axial direction.

9. The stent assembly method of claim 7, wherein in the state that the assembly part and the stent are hooked, the step of accommodating the sheath core tube and the stent together into the outer sheath tube comprises: keeping the sheath core tube and the stent stationary, and pushing the outer sheath tube in a direction close to the sheath core tube and the stent so that the sheath core tube and the stent are accommodated in the outer sheath tube.

10. The stent assembly method of claim 7, wherein the stent comprises a supporting framework and a covered film that surrounds the supporting framework; in a radially compressed state, a plurality of folds are formed on the covered film; and a free end of the assembly part extends into the folds.
